(19)

Europäisches
Patentamt
European
Patent Office
Office européen
des brevets

(11) **EP 3 173 016 A1**

(12) **EUROPEAN PATENT APPLICATION**

(43) Date of publication:
**31.05.2017 Bulletin 2017/22**

(21) Application number: **16200173.9**

(22) Date of filing: **23.11.2016**

(51) Int Cl.:
*A61B 5/00* (2006.01)      *A61B 5/042* (2006.01)
*A61B 18/12* (2006.01)     *A61B 18/14* (2006.01)
*A61B 5/024* (2006.01)     *A61B 18/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **24.11.2015 US 201562259370 P
01.11.2016 US 201615340547**

(71) Applicant: **Biosense Webster (Israel) Ltd.
2066717 Yokneam (IL)**

(72) Inventors:
• **Levin, Michael
2066717 Yokneam (IL)**
• **Reuveni, Avi
2066717 Yokneam (IL)**

(74) Representative: **Carpmaels & Ransford LLP
One Southampton Row
London WC1B 5HA (GB)**

(54) **ENHANCED SAFETY METHOD AND SYSTEM FOR DIGITAL COMMUNICATION USING TWO AC COUPLING WIRES**

(57)      Provided are a method and apparatus for performing medical catheterization using electrical power circuitry disposed outside a medical catheter and remote internal circuitry disposed within the medical catheter. At least one of the power circuitry and the internal circuitry is isolated from electrical ground. Exactly two wires connect the power circuitry to the internal circuitry and a signal generator is provided for generating an alternating carrier that is communicated from the power circuitry to the internal circuitry via the wires. Decoders are disposed in the power circuitry and the internal circuitry, and a transceiver performs half-duplex data communication between the power circuitry and the internal circuitry by alternately modulating the carrier voltage amplitude in one of the power circuitry and the internal circuitry and decoding the modulated carrier voltage amplitude in another of the power circuitry and the internal circuitry.

**EP 3 173 016 A1**

## Description

**CROSS-REFERENCE TO RELATED APPLICATIONS**

This Application claims the benefit of U.S. Provisional Application No. 62/259,370, filed 24 November 2015, which is herein incorporated by reference.

**FIELD**

The presently described subject matter relates to transferring non-mechanical forms of energy to or from the body. More particularly, the described subject matter relates to transferring radiofrequency energy into the heart for ablation.

**BACKGROUND**

Mapping of electrical potentials in the heart is now commonly performed, using cardiac catheters comprising electrophysiological sensors for mapping the electrical activity of the heart. Typically, time-varying electrical potentials in the endocardium are sensed and recorded as a function of position inside the heart, and then used to map a local electrogram or local activation time.

When conduction abnormalities, such as atrial fibrillation are present, radiofrequency (RF) ablation of the heart is a procedure that is widely used to correct problematic cardiac conditions. The procedure typically involves insertion of a catheter having an electrode into the heart, and ablating selected regions within the heart with RF energy transmitted via the electrode.

**SUMMARY**

Safety requirements can be limiting factors in miniaturization of devices for intra-body applications. For example, catheters used for electrophysiological procedures have various sensors in their distal portions, while processing of the signals from the sensors can occurs proximally. However, providing signal processing capabilities in the distal portion of the catheter can improve the quality of the electrophysiological data and measurements. Signal processing circuitry requires electrical power to be supplied to the distal portion of the catheter. If mechanical failure of the catheter shaft or a short circuit in the power wires supplying the signal processing circuitry were to occur, the subject could experience an electrical shock. The presently described subject matter is directed to methods and systems that provide electrical safety in devices for intra-body applications.

The presently described subject matter is directed to a method, comprising disposing electrical power circuitry outside a medical catheter; disposing remote internal circuitry within the medical catheter; isolating at least one of the power circuitry and the internal circuitry from electrical ground; connecting the power circuitry to the internal circuitry by two wires, for example, by exactly two wires; and communicating an alternating carrier from the power circuitry to the internal circuitry via the two wires. The method may further comprise performing half-duplex data communication between the power circuitry and the internal circuitry by, for example, alternately modulating the carrier voltage amplitude with one of the power circuitry and the internal circuitry and decoding the modulated carrier voltage amplitude with another of the power circuitry and the internal circuitry.

According to one aspect of the presently described method, the power circuitry can comprise a transceiver for modulating the carrier voltage amplitude and a decoder for demodulating the carrier voltage amplitude.

According to a further aspect of the presently described method, the internal circuitry can comprise a decoder for demodulating the carrier voltage amplitude.

Yet another aspect of the method can comprise obtaining and processing data in the internal circuitry from sensors in the catheter and modulating the carrier voltage amplitude according to the processed data for communication a decoder to the power circuitry.

According to still another aspect of the method alternately modulating the carrier voltage amplitude is performed with a first switch in the power circuitry and with a second switch in the internal circuitry to vary first and second resistances across the alternating carrier, respectively.

According to certain embodiments of the presently described subject matter an apparatus, further provided is an apparatus comprising a medical catheter; electrical power circuitry disposed outside the catheter; and remote internal circuitry disposed within the catheter. At least one of the power circuitry and the internal circuitry is isolated from electrical ground. The apparatus may further include two wires, for example, exactly two wires, connecting the power circuitry to the internal circuitry, and a signal generator for generating an alternating carrier that is communicated from the power circuitry to the internal circuitry via the wires. Decoders are disposed in the power circuitry and the internal circuitry, and a transceiver performs half-duplex data communication between the power circuitry and the internal circuitry. The transceiver is operative for alternately modulating the carrier voltage amplitude in one of the power circuitry and the internal circuitry and decoding the modulated carrier voltage amplitude in the decoder of another of the power circuitry and the internal circuitry.

## BRIEF DESCRIPTION OF THE SEVERAL VIEWS OF THE DRAWINGS

For a better understanding of the presently described subject matter, reference is made to the detailed description of the presently described subject matter, by way of example, which is to be read in conjunction with the following drawings, wherein like elements are given like reference numerals, and wherein:

Fig. 1 is a pictorial illustration of a system, which is constructed and operative in accordance with a disclosed embodiment of the presently described subject matter;

Fig. 2 is an electrical schematic of an embodiment of a system for digital communication in accordance with an embodiment of the presently described subject matter; and

Fig. 3 is a flow chart illustrating a sequence of operations using the system shown in Fig. 2 in accordance with an embodiment of the presently described subject matter.

## DETAILED DESCRIPTION

In the following description, numerous specific details are set forth in order to provide a thorough understanding of the various principles of the presently described subject matter. It will be apparent to one skilled in the art, however, that not all these details are necessarily needed for practicing the presently described subject matter. In this instance, well-known circuits, control logic, and the details of computer program instructions for conventional algorithms and processes have not been shown in detail in order not to obscure the general concepts unnecessarily.

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

### Overview.

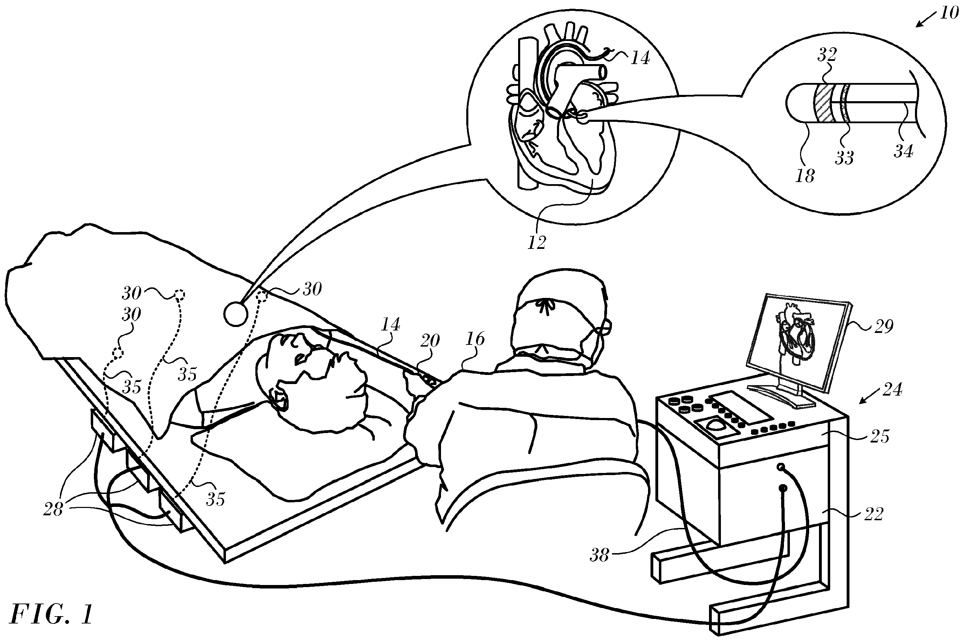

Turning now to the drawings, reference is initially made to Fig. 1, which is a pictorial illustration of a system 10 for evaluating electrical activity and performing ablative procedures on a heart 12 of a living subject, which is constructed and operative in accordance with a disclosed embodiment of the presently described subject matter. The system comprises a catheter 14, which is percutaneously inserted by an operator 16 through the patient's vascular system into a chamber or vascular structure of the heart 12. The operator 16, who is typically a physician, brings the catheter's distal tip 18 into contact with the heart wall, for example, at an ablation target site.

Electrical activation maps may be prepared, according to the methods disclosed in U.S. Patent Nos. 6,226,542, and 6,301,496, and in commonly assigned U.S. Patent No. 6,892,091, whose disclosures are herein incorporated by reference. One commercial product embodying elements of the system 10 is available as the CARTO® 3 System, available from Biosense Webster, Inc., 3333 Diamond Canyon Road, Diamond Bar, CA 91765. This system may be modified by those skilled in the art to embody the principles of the presently described subject matter.

Areas determined to be abnormal, for example by evaluation of the electrical activation maps, can be ablated by application of thermal energy, e.g., by passage of radiofrequency electrical current through wires in the catheter to one or more electrodes at the distal tip 18, which apply the radiofrequency energy to the myocardium. The energy is absorbed in the tissue, heating it to a point (typically about 50°C) at which it permanently loses its electrical excitability. When successful, this procedure creates non-conducting lesions in the cardiac tissue, which disrupt the abnormal electrical pathway causing the arrhythmia. The principles of the presently described subject matter can be applied to different heart chambers to diagnose and treat many different cardiac arrhythmias.

The catheter 14 can comprise a handle 20, having suitable controls on the handle to enable the operator 16 to steer, position and orient the distal end of the catheter as desired for the ablation. To aid the operator 16, the distal portion of the catheter 14 contains position sensors (not shown) that provide signals to a processor 22, located in a console 24. The processor 22 may fulfill several processing functions as described below.

Ablation energy and electrical signals can be conveyed to and from the heart 12 through one or more ablation electrodes 32 located at or near the distal tip 18 via cable 34 to the console 24. Pacing signals and other control signals may be conveyed from the console 24 through the cable 34 and the electrodes 32 to the heart 12. Sensing electrodes 33, also connected to the console 24 are disposed between the ablation electrodes 32 and have connections to the cable 34.

Wire connections 35 link the console 24 with body surface electrodes 30 and other components of a positioning sub-system for measuring location and orientation coordinates of the catheter 14. The processor 22 or another processor (not shown) may be an element of the positioning subsystem. The electrodes 32 and the body surface electrodes 30 may be used to measure tissue impedance at the ablation site as taught in U.S. Patent No. 7,536,218, issued to Govari et al., which is herein incorporated by reference. A temperature sensor (not shown), including for example, a thermocouple or thermistor, may be mounted on or near each of the electrodes 32.

The console 24 may contain one or more ablation power generators 25. The catheter 14 may be configured to conduct ablative energy to the heart using any known ablation technique, including for example, but not limited to, radiofrequency energy, ultrasound energy, and laser-produced light energy. Such methods are disclosed in commonly assigned U.S. Patent Nos. 6,814,733, 6,997,924, and 7,156,816, both of which are herein incorporated by reference.

In one embodiment, the positioning subsystem can comprise a magnetic position tracking arrangement that determines the position and orientation of the catheter **14** by generating magnetic fields in a predefined working volume and sensing these fields at the catheter, using field generating coils **28**. The positioning subsystem is described in U.S. Patent No. 7,756,576, which is hereby incorporated by reference, and in the above-noted U.S. Patent No. 7,536,218.

As noted above, the catheter **14** is coupled to the console **24,** which enables the operator **16** to observe and regulate the functions of the catheter **14**. Console **24** includes a processor, preferably a computer with appropriate signal processing circuits. The processor is coupled to drive a monitor **29**. The signal processing circuits typically receive, amplify, filter, and digitize signals from the catheter **14,** including, for example, signals generated by sensors, including but not limited to, electrical, temperature, and contact force sensors, and a plurality of location sensing electrodes (not shown) located distally in the catheter **14**. The digitized signals are received and used by the console **24** and the positioning system to compute the position and orientation of the catheter **14,** and to analyze the electrical signals from the electrodes.

Typically, the system **10** includes other elements, which are not shown in the figures for the sake of simplicity. For example, the system **10** may include an electrocardiogram (ECG) monitor, coupled to receive signals from one or more body surface electrodes, in order to provide an ECG synchronization signal to the console **24**. As mentioned above, the system **10** may also include a reference position sensor, either on an externally-applied reference patch attached to the exterior of the subject's body, or on an internally-placed catheter, which is inserted into the heart **12** maintained in a fixed position relative to the heart **12**. Conventional pumps and lines for circulating liquids through the catheter **14** for cooling the ablation site can be provided. The system **10** may receive image data from an external imaging modality, such as an MRI unit or the like and includes image processors that can be incorporated in or invoked by the processor **22** for generating and displaying images.

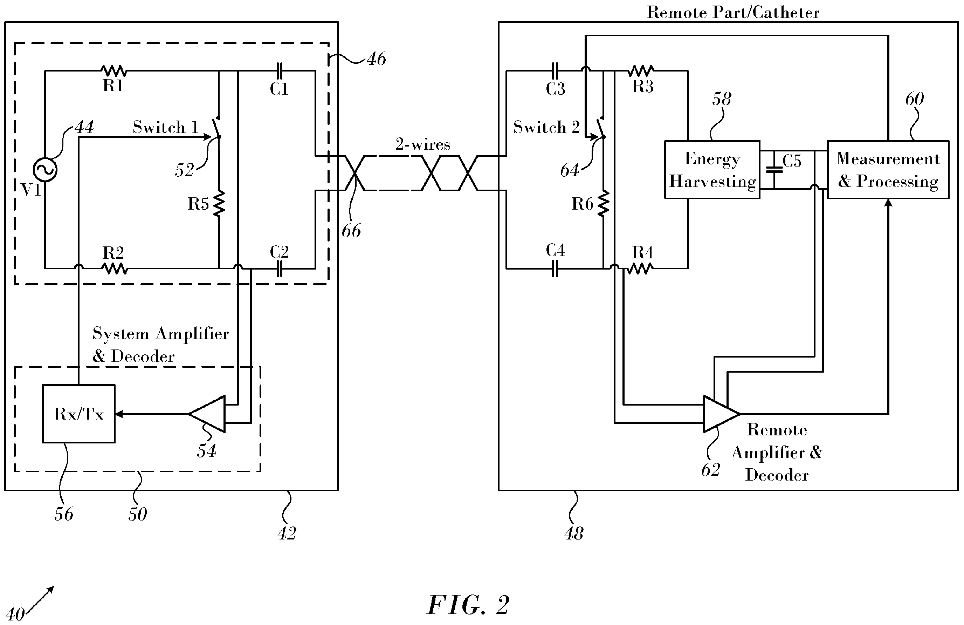

Reference is now made to **Fig. 2,** which is an electrical schematic of an embodiment of a system for digital communication in a catheter using two alternating current (AC) coupling wires in accordance with an embodiment of the presently described subject matter. The components shown are dimensioned to an intra-body catheter.

A system **40** comprises electrical power circuitry **42,** which is can be located outside the catheter, for example, in the console **24 (Fig. 1)**. Power circuitry **42** comprises an alternating current signal generator **44** connected in a power supply circuit **46**. The signal generator **44** generates a carrier frequency in the range of tens or hundreds of kHz. The AC current passes through a network comprising resistors **R1**, **R2** and capacitors **C1**, **C2**. The AC current is used both as an electrical energy source for remote circuitry **48** and as the carrier frequency for information transfer between the power circuitry **42** and remote circuitry **48**.

Power circuitry **42** includes a signal processing module **50,** which controls a switch **52** (ON/OFF) to modulate the carrier frequency. The signal processing module **50** includes an amplifier **54** and a transceiver **56**. The amplifier **54** receives and decodes or demodulates signals that are received from internal remote circuitry **48**. The transceiver **56** handles communications that are directed to the remote circuitry **48**.

The remote circuitry **48** comprises an energy harvesting component **58,** a measurement and processing component **60** and an amplifier and decoder **62** for demodulating the carrier voltage amplitude. The energy harvesting component **58,** which can be model LTC3331 from Linear Technology, converts the AC voltage at its input to a DC voltage and charges the storage capacitor **C5** to a constant value that can be in the range of 3 to 10 volts direct current (VDC). The measurement and processing component **60** and amplifier and decoder **62** are switched in when the DC voltage on the capacitor **C5** reaches a predetermined value by switch **64**.

As noted above, the remote circuitry **48** is remote from the power circuitry **42**. The power circuitry **42** and the remote circuitry **48** are connected by a wire pair **66**. The wire pair **66** may be implemented by a twisted pair that reduces sensitivity to external magnetic fields.

**Operation.**

An AC carrier current produced by signal generator **44** passes through resisters **R3, R4** and capacitors **C3, C4** in the power circuitry **42;** then through wire pair **66** into the remote circuitry **48**. Data communication between the power circuitry **42** and remote circuitry **48** is implemented by carrier voltage amplitude modulation. The signal generator **44** together with the resistors **R1** and **R2** act as the current source and the voltage across the wires of the wire pair **66** depends on the impedance across the two wires.

When both switches **52, 64** are open, i.e., in an OFF state, and the impedance of the capacitors **C1** and **C2** at the carrier frequency is much less than the values of **R1** and **R2**, the transmission (Tx) voltage between the wires assumes a first value.:

$$Tx = V1 * [Rc/(R1+R2+Rc)],$$

where V1 is the output voltage of the signal generator **44** and Rc is the impedance of the parasitic capacitance and the load of the remote circuitry **48** at the carrier frequency.

As noted above, the signal processing module **50** modulates the carrier voltage amplitude by varying switch **52** between open and closed positions. Signal processing module 50 influences only switch 52 and the remote circuitry **48** influences switch **64**. When switch **52** is closed and switch **64** is open, the Tx voltage between across the wire pair 66 assumes a second value:

$$Tx=V1*(Rc/R1+R2+Rc+R6.$$

When switch **52** is opened and switch **64** is closed the Tx voltage between across the wire pair **66** assumes a third value:

$$Tx=V1*(Rc/R1+R2+Rc+R5).$$

The amplifier and decoder **62** in the remote circuitry **48** receives the modulated carrier voltage and demodulates the information that is embedded in the input signal.

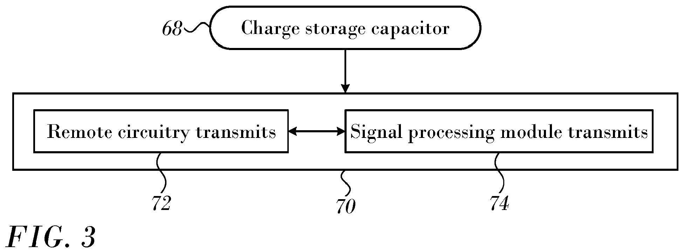

Reference is now made to **Fig. 3,** which is a flow chart illustrating a sequence of operations using the system **40 (Fig. 2),** in accordance with an embodiment of the presently described subject matter. The process steps are shown in a particular linear sequence for clarity of presentation. However, it will be evident that many of them can be performed in parallel, asynchronously, or in different orders. Those skilled in the art will also appreciate that a process could alternatively be represented as a number of interrelated states or events, e.g., in a state diagram. Moreover, not all illustrated process steps may be required to implement the method.

At initial step **68** switches **52, 64** are both opened. The voltage at energy harvesting component **58** is maximal and it charges the storage capacitor **C5.** When the storage capacitor is charged, the remote unit **48** is ready to work.

Next, communication step **70** is performed, which comprises two steps **72, 74,** which are performed in alternation, i.e., the communication is half-duplex. Any suitable communications protocol may be used:

In step **72** the remote circuitry **48** transmits data to the signal processing module **50,** modulating a carrier voltage by opening and closing switch **64**. Switch **52** remains open during step **72**.

In step **74** the signal processing module **50** transmits commands to the remote circuitry **48,** modulating the carrier voltage by opening and closing switch **52**. Switch **64** remains open during step **74**.

It should be noted that the remote circuitry **48** is fully isolated from the power circuitry **42**. There is no common ground connection between the two components. If a short between the wires of the wire pair **66** should occur, the patient would be exposed only to a low voltage. Disconnection would result in a higher voltage than normal but still within a low range, so that patient safety would not be compromised. For example, if the generator's output voltage is no more than 2V and resistors **R1, R2** are in the range of 50kΩ, the maximum current through the patient's body would be 2V/100kΩ=20uA. In this regard, it may be noted that the maximum allowable current through the patient's body in a single fault condition according to the standard IEC60601-1 is 50uA.

It will be appreciated by persons skilled in the art that the presently described subject matter is not limited to what has been particularly shown and described hereinabove. Rather, the scope of the present presently described subject matter includes both combinations and sub-combinations of the various features described hereinabove, as well as variations and modifications thereof that are not in the prior art, which would occur to persons skilled in the art upon reading the foregoing description.

**Claims**

1. A method, comprising the steps of:

   disposing electrical power circuitry outside a medical catheter;
   disposing remote internal circuitry within the medical catheter;
   isolating the power circuitry and the internal circuitry from electrical ground;
   connecting the power circuitry to the internal circuitry by exactly two wires;
   communicating an alternating carrier from the power circuitry to the internal circuitry via the wires, the carrier having a carrier voltage amplitude; and
   performing half-duplex data communication between the power circuitry and the internal circuitry by alternately modulating the carrier voltage amplitude with one of the power circuitry and the internal circuitry and decoding the modulated carrier voltage amplitude with another of the power circuitry and the internal circuitry.

2. The method according to claim 1, wherein the power circuitry comprises a transceiver for modulating the carrier voltage amplitude and a decoder for demodulating the carrier voltage amplitude.

3. The method according to claim 1, wherein the internal circuitry comprises a decoder for demodulating the carrier voltage amplitude.

4. The method according to claim 1, further comprising:

in the internal circuitry obtaining and processing data from sensors in the medical catheter; and modulating the carrier voltage amplitude according to the processed data for communication to the power circuitry.

5. The method according to claim 1, wherein alternately modulating the carrier voltage amplitude is performed with a first switch in the power circuitry and with a second switch in the internal circuitry to vary first and second resistances across the alternating carrier, respectively.

6. An apparatus, comprising:

     a medical catheter
     electrical power circuitry disposed outside the medical catheter;
     remote internal circuitry disposed within the medical catheter, the power circuitry and the internal circuitry being isolated from an electrical ground;
     exactly two wires connecting the power circuitry to the internal circuitry;
     a signal generator for generating an alternating carrier, the carrier being communicated from the power circuitry to the internal circuitry via the wires, the carrier having a carrier voltage amplitude; and
     a respective decoder in the power circuitry and the internal circuitry; and
     a transceiver for performing half-duplex data communication between the power circuitry and the internal circuitry, the transceiver operative for alternately modulating the carrier voltage amplitude in one of the power circuitry and the internal circuitry and decoding the modulated carrier voltage amplitude in the decoder of another of the power circuitry and the internal circuitry.

7. The apparatus according to claim 6, wherein the decoder is operative for demodulating the carrier voltage amplitude.

8. The apparatus according to claim 6, further comprising sensors in the catheter, the internal circuitry operative for obtaining and processing data from and modulating the carrier voltage amplitude according to the processed data for communication to the power circuitry.

9. The apparatus according to claim 6, further comprising:

     a first switch in the power circuitry and
     a second switch in the internal circuitry, the first switch and the second switch operative to vary first and second resistances across the signal generator, respectively.

*FIG. 1*

EP 3 173 016 A1

FIG. 2

68 ⟶ ( Charge storage capacitor )

Remote circuitry transmits ⟷ Signal processing module transmits

72     70     74

*FIG. 3*

Europäisches
Patentamt

European
Patent Office

Office européen
des brevets

# EUROPEAN SEARCH REPORT

Application Number

EP 16 20 0173

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/194753 A1 (DEKKER RONALD [NL] ET AL) 10 July 2014 (2014-07-10)<br>* abstract; figures 1-3 *<br>* paragraphs [0008] - [0011], [0019] - [0022], [0025] * | 1-9 | INV.<br>A61B5/00<br>A61B5/042<br>A61B18/12<br>A61B18/14 |
| A | WO 2015/000500 A1 (CATHVISION APS [DK]) 8 January 2015 (2015-01-08)<br>* abstract; figures 1-5 *<br>* page 3, line 15 - page 4, line 32 *<br>* page 6, line 28 - page 7, line 32 *<br>* page 15, line 15 - page 22, line 10 * | 1-9 | ADD.<br>A61B5/024<br>A61B18/00 |
| A | US 2012/195078 A1 (LEVIN MICHAEL [IL] ET AL) 2 August 2012 (2012-08-02)<br>* abstract; figures 1,2 *<br>* paragraphs [0005] - [0007], [0009], [0012], [0018] - [0030] * | 1-9 | |
| A | US 2015/088115 A1 (SMITH ROBERT B [US]) 26 March 2015 (2015-03-26)<br>* figures 1-8 *<br>* paragraphs [0038], [0053] - [0055], [0061] * | 1-9 | TECHNICAL FIELDS SEARCHED (IPC)<br>A61B<br>A61N |
| A | EP 2 587 275 A1 (KONINKL PHILIPS ELECTRONICS NV [NL]; PHILIPS INTELLECTUAL PROPERTY [DE] 1 May 2013 (2013-05-01)<br>* abstract; figures 1-4 *<br>* paragraphs [0022] - [0024] * | 1-9 | |

The present search report has been drawn up for all claims

| Place of search | Date of completion of the search | Examiner |
|---|---|---|
| The Hague | 5 April 2017 | Carta, Riccardo |

EPO FORM 1503 03.82 (P04C01)

## ANNEX TO THE EUROPEAN SEARCH REPORT
## ON EUROPEAN PATENT APPLICATION NO.

EP 16 20 0173

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2017

| Patent document cited in search report | | Publication date | Patent family member(s) | | Publication date |
|---|---|---|---|---|---|
| US 2014194753 | A1 | 10-07-2014 | NONE | | |
| WO 2015000500 | A1 | 08-01-2015 | NONE | | |
| US 2012195078 | A1 | 02-08-2012 | AU | 2012200418 A1 | 16-08-2012 |
| | | | CA | 2765976 A1 | 01-08-2012 |
| | | | CN | 102629750 A | 08-08-2012 |
| | | | EP | 2481369 A1 | 01-08-2012 |
| | | | JP | 2012157702 A | 23-08-2012 |
| | | | US | 2012195078 A1 | 02-08-2012 |
| US 2015088115 | A1 | 26-03-2015 | EP | 2851017 A1 | 25-03-2015 |
| | | | US | 2015088115 A1 | 26-03-2015 |
| EP 2587275 | A1 | 01-05-2013 | CN | 101652672 A | 17-02-2010 |
| | | | EP | 2097762 A1 | 09-09-2009 |
| | | | EP | 2587275 A1 | 01-05-2013 |
| | | | JP | 5404416 B2 | 29-01-2014 |
| | | | JP | 2010529863 A | 02-09-2010 |
| | | | US | 2010013484 A1 | 21-01-2010 |
| | | | WO | 2008078294 A1 | 03-07-2008 |

EPO FORM P0459

For more details about this annex : see Official Journal of the European Patent Office, No. 12/82

**EP 3 173 016 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62259370 A
- US 6226542 B
- US 6301496 B
- US 6892091 B
- US 7536218 B, Govari
- US 6814733 B
- US 6997924 B
- US 7156816 B
- US 7756576 B